# EUROPÄISCHE PATENTANMELDUNG

(11) **EP 1 285 959 A1**
(43) Veröffentlichungstag der Anmeldung: **26.02.2003**
(21) Anmeldenummer: 01120067.2
(22) Anmeldetag: 21.08.2001
(51) Int. Cl.: C12M 1/107

(54) **Bioreaktorsystem zur Nutzung der Wärmeentwicklung biochemischer Reaktionen**

(71) Anmelder: Höfer Bioreact GmbH, 53115 Bonn (DE)
(72) Erfinder: Hölker, Udo, 53639 Königswinter/Rauschendorf (DE)
(74) Vertreter: Helbing, Jörg, Dr. Dipl.-Chem.

(57) **Zusammenfassung**

Die vorliegende Erfindung betrifft ein Bioreaktorsystem zur Nutzung der mikrobiellen Wärmeentwicklung bei der Verstoffwechselung organischer Substrate und ein kontinuierliches Verfahren zum mikrobiellen Abbau organischer Substrate zur Gewinnung thermischer Energie.

## Beschreibung

Die vorliegende Erfindung betrifft ein Bioreaktorsystem zur Nutzung der mikrobiellen Wärmeentwicklung bei der Verstoffwechselung organischer Substrate und ein kontinuierliches Verfahren zum mikrobiellen Abbau organischer Substrate zur Gewinnung thermischer Energie.

### Hintergrund der Erfindung

Die Metabolisierung von organischen Substraten durch Mikroorganismen erfolgt durch sukzessive Oxidation des eingesetzten Substrates bis hin zum CO₂. Das Substrat wird dabei zur Speicherung von chemischer Energie in Form von ATP und zum Aufbau der Zellmasse verwendet. Dabei geht bei jeder chemischen Reaktion ein Teil der inneren Energie (H) als Entropie S (Wärme) verloren. Bei Fermentationen in denen Mikroorganismen aus einem Substrat ein gewünschtes Endprodukt herstellen, ist die Produktion von Wärme eine unerwünschte Begleiterscheinung. Überschüssige Wärme muss aus dem Prozess durch Kühlsysteme abgeleitet werden.

Es ist bekannt, dass Bioreaktoren zur Produktion von thermischer Energie verwendet werden können. Dies geschieht in den meisten Fällen indirekt. Zunächst wird das aufbereitete Substrat in Wasser gelöst und von den Mikroorganismen anaerob zu Methan reduziert. Anschließend wird das Methan zu CO₂ verbrannt und die frei werdende thermische Energie genutzt. Auch aerobe Versuche zur Kompostierung von organischen Substraten in einer der Prozesssteuerung und Regelung nicht zugänglichen Miete wurden durchgeführt.

Eine direkte Nutzung, der bei jeder chemischen (biologischen) Reaktion frei werdenden Wärme, würde eine nachhaltige Entlastung der Umwelt und der globalen Ressourcen bedeuten.

Die Aufgabe der Erfindung bestand darin, zentrale und dezentrale Bioreaktoren zur Verfügung zu stellen, die in jedem Haushalt installiert werden können. Dabei soll eine größtmögliche konstante Wärme produziert werden, diese über einen Wärmetauscher abgeleitet und zur Warmwasserproduktion sowie zur Elektrizitätsgewinnung genutzt werden. Insbesondere soll die thermische Fermentation mit hohen Feststoffgehalten durchgeführt werden, um organische Haushaltsabfälle verwerten zu können und um möglichst wenig Wasser zu verbrauchen.

### Kurzbeschreibung der Erfindung

Die Erfindung betrifft somit
(1) ein Bioreaktorsystem zur Nutzung der Wärmeentwicklung biochemischer Reaktionen, insbesondere zur Warmwasser- und Stromproduktion, mit einem Bioreaktor (I,II) zum im Wesentlichen aeroben mikrobiellen Abbau organischer Substrate, einem Bioreaktor (III) zum im Wesentlichen anaeroben mikrobiellen Abbau organischer Substrate, wobei die Bioreaktoren (I,II,III) nacheinander geschaltet sind, so dass den nachgeschalteten Bioreaktoren (II,III) das Abbausubstrat des vorgeschalteten Bioreaktors (I) bzw. (II) zugeführt wird, und zumindest einem Wärmetauscher zur Wärmeabfuhr aus den Bioreaktoren (I,II,III); und
(2) ein kontinuierliches Verfahren zum mikrobiellen Abbau organischer Substrate zur Gewinnung thermischer Energie, umfassend wenigstens einen im Wesentlichen aeroben und einen im Wesentlichen anaeroben Verfahrensschritt in nacheinander geschalteten diskreten Reaktionsgefäßen.

Mit Hilfe des erfindungsgemäßen Bioreaktorsystems ist es insbesondere möglich, organische Haushaltsabfälle mikrobiell abzubauen und hierbei Energie für die Warmwasseraufbereitung und/oder die Stromproduktion zu erzeugen. Es findet somit nicht nur eine erhebliche Reduzierung des organischen Mülls durch mikrobiellen Abbau, sondern auch eine Nutzung der hierbei entstehenden Wärme statt. Zur Nutzung der Wärme ist erfindungsgemäß ein Wärmetauscher vorgesehen. Der Wärmetauscher kann mit den einzelnen Bioreaktoren gekoppelt sein. Das Vorsehen eines einzigen Wärmetauschers oder eines gemeinsamen Wärmetauschers für zwei oder mehr Bioreaktoren hat zur Folge, dass die Temperaturen dieser Bioreaktoren miteinander gekoppelt sind. Es ist somit zusätzlich zur Wärmeabfuhr auch sichergestellt, dass die Temperatur eines einzelnen Bioreaktors nicht unter eine gewünschte Temperatur absinkt.

Bei einer besonders bevorzugten Ausführungsform sind zwei im wesentlichen aerobe Bioreaktoren und ein im Wesentlichen anaerober Bioreaktor vorgesehen. Die drei Bioreaktoren sind nacheinander geschaltet, wobei zuerst die beiden aerob arbeitenden Bioreaktoren und dann der anaerob arbeitende Bioreaktor vorgesehen ist. Vorzugsweise handelt es sich bei dem ersten Bioreaktor um einen Feststoffbioreaktor, dem vorzugsweise durch eine Düsenanordnung Sauerstoff zugeführt wird. Bei dem zweiten im Wesentlichen aerob arbeitenden Bioreaktor werden die organischen Substrate vorzugsweise mit Wasser versetzt, so dass eine zähflüssige förderbare Masse mit freier Wasserphase entsteht. Es handelt sich bei dem zweite Bioreaktor vorzugsweise um einen Wirbelschicht-Bioreaktor, bei dem die organischen Substrate auf Grund des Medienstroms in Lösung gehalten werden. Das in dem zweiten Bioreaktor vorzugsweise durch eine Entnahme im oberen Bereich und ein Zuführen im unteren Bereich kontinuierlich geförderte Medium wird vorzugsweise durch eine Belüftungs- und Regulierungseinrichtung geführt. In dieser erfolgt vorzugsweise die Anreicherung des Mediums mit Sauerstoff sowie die Regulierung des pH-Werts. Besonders bevorzugt ist es, hierzu die Abgase bzw. die Abluft aus dem ersten Bioreaktor zu nutzen. Ggf. können aus der Belüftungs- und Regulierungseinrichtung auch Abgase abgeführt werden. Da es sich hierbei im Allgemeinen um brennbare Gase handelt, werden diese vorzugsweise einer Verbrennungseinrichtung zugeführt. Die Verbrennungseinrichtung kann wiederum zur Energiegewinnung, insbesondere zur Warmwasseraufbereitung oder Stromerzeugung, genutzt werden.

Besonders bevorzugt ist die Verwendung der erfindungsgemäßen Vorrichtung zur Durchführung des erfindungsgemäßen Verfahrens. Im folgenden wird die Erfindung anhand einer bevorzugten Ausführungsform des Bioreaktorsystems näher erläutert.

### Figur

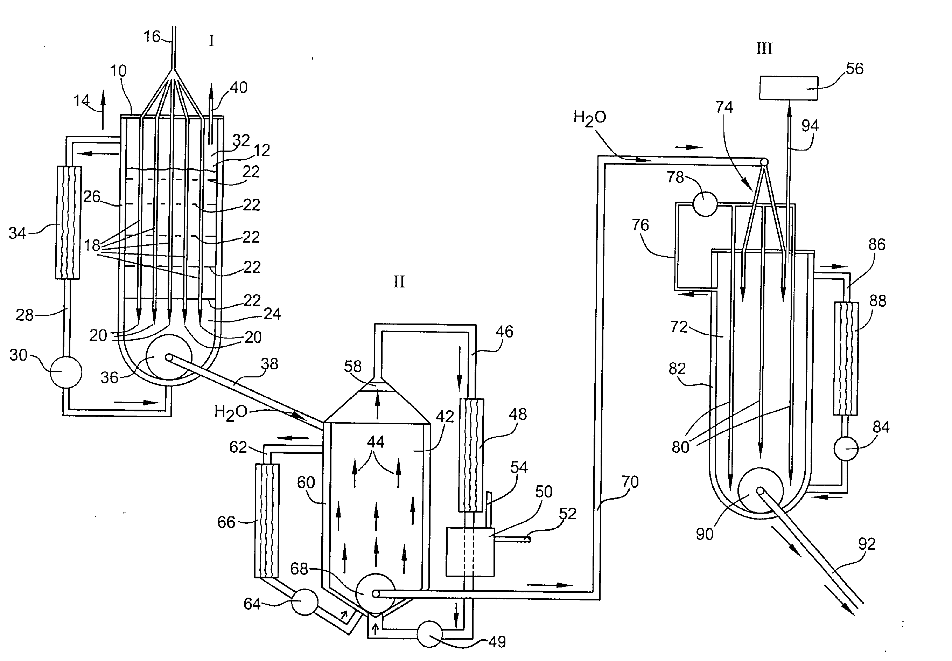

Die Figur zeigt eine Prinzipskizze einer bevorzugten Ausführungsform des Bioreaktorsystems.

### Ausführliche Beschreibung der Erfindung

Ein vorangeschalteter erster Bioreaktor (I) kann mit hohen Feststoffgehalten, z.B. organischen Abfällen, die nicht zusätzlich aufbereitet werden müssen, betrieben werden und ist bevorzugt für Pilze geeignet. Die Fermentationsgeschwindigkeit und damit die konstante Wärmeentwicklung im Bioreaktor wird über die gezielte Versorgung der Mikroorganismen mit Sauerstoff gewährleistet.

Der vorgeschlagene Bioreaktor erlaubt die kontinuierliche Nutzung der bei der Veratmung von organischen Materialien durch Mikroorganismen freigesetzten Wärme.

Der erste Bioreaktor I, bei dem es sich um einen im Wesentlichen aerob arbeitenden Bioreaktor handelt, weist einen mit einem Deckel 10 verschlossenen Reaktionsbehälter 12 auf. Durch Öffnen des Deckels 10 und beispielsweise durch ein mechanisches Verfahren des Deckels 10 in Richtung des Pfeils 14 können in den Reaktionsbehälter 12 organische Substrate mit hohem Feststoffgehalt zugeführt werden. Zusätzlich oder anstatt des Deckels 10 kann auch eine Zerkleinerungseinrichtung zum Zerkleinern der organischen Abfälle vorgesehen sein, die über ein Rohr o.dgl. mit einer Öffnung des Reaktionsbehälters 12 verbunden ist, um die Substrate zuzuführen.

Ferner weist der erste Bioreaktor I eine Düsenanordnung 16 mit mehreren vertikal verlaufenden Düsen 18 auf. Durch die Düsen 18, die vorzugsweise regelmäßig über den gesamten Behälter 12 verteilt sind, kann dem Behälter 12 Sauerstoff zugeführt werden. Hierzu ist die Düsenanordnung 16 vorzugsweise mit einem Kompressor verbunden. Die einzelnen Düsen 18 der Düsenanordnung 16 können in unterschiedlicher Höhe des Behälters 12 enden. Vorzugsweise erstrecken sie sich durch den gesamten Behälter, so dass die unteren Düsenöffnungen 20 im unteren Bereich des Behälters 12 angeordnet sind. Jede einzelne Düse 18 kann mehrere Luftaustrittsöffnungen, die über die Höhe des Behälters 12 verteilt sind, aufweisen. Durch die Düsenanordnung erfolgt ein Belüften und Durchmischen der in dem Behälter 12 befindlichen Substrate.

Innerhalb des Behälters 12 sind mehrere vorzugsweise horizontal angeordnete Siebe 22 vorgesehen, wobei sich vorzugsweise die Maschenweite der Siebe 22 in Förderrichtung verringert. Bei dem dargestellten Ausführungsbeispiel erfolgt die Förderung der Substrate in dem ersten Bioreaktor I in vertikaler Richtung von oben nach unten auf Grund von Schwerkraft. Die Maschenweite der Siebe 22 nimmt somit von oben nach unten ab.

Die Düsen 18 der Düsenanordnung 16 erstrecken sich vorzugsweise in vertikaler Richtung und durchdringen die Siebe 22. Insbesondere erfolgt durch die Düsenanordnung eine Zufuhr von mit Wasser gesättigter Druckluft in den Reaktionsbehälter 12. Mit Hilfe der Menge des eingeleiteten Sauerstoffs kann die Reaktionsgeschwindigkeit der in dem Behälter 12 stattfindenden Oxidationsprozesse und damit die bei der Fermentation freiwerdende Wärmemenge gesteuert werden.

Zersetzte, d.h. oxidierte, Substrate sedimentieren und fallen durch die Siebe 22 nach unten. Durch die Siebe 22 ist sichergestellt, dass in einem unteren Bereich 24 des Behälters 12 nur Substrat einer durch die Maschenweite des unteren Siebes 22 definierten Größe vorkommt.

Zur Wärmeabfuhr ist der Reaktionsbehälter 12 doppelwandig ausgebildet, so dass in einem Zwischenraum 26 ein geeignetes Wärmetransportmedium, wie beispielsweise Wasser, vorgesehen sein kann. Zum Umwälzen des Wärmetransportmediums ist der doppelwandige Behälter 12 mit einer Rohrleitung 28 verbunden, in der eine Pumpe 30 angeordnet ist. Das Wärmetransportmedium wird aus einem oberen Bereich 32 des Behälters 12 entnommen und im unteren Bereich 24 des Behälters 12 diesem wieder zugeführt. Mittels eines in der Rohrleitung 28 angeordneten Wärmetauschers 34 wird dem Wärmetransportmedium Wärme entnommen. Diese Wärme kann zur Aufbereitung von Warmwasser oder zur Gewinnung von Strom genutzt werden. Anstelle eines außerhalb des Reaktionsbehälters 12 angeordneten Wärmetauschers 34 kann auch ein innerhalb des Reaktionsbehälters 12 vorgesehener Wärmetauscher vorgesehen sein.

Die in den Bereich 24 des Behälters 12 gelangenden Substrate werden über eine Fördereinrichtung 36, wie beispielsweise eine Schraubenschnecke, zu einem zweiten Bioreaktor II über eine Rohrleitung 38 transportiert.

Ferner entsteht in dem Reaktionsbehälter 12 bei der Fermentation der organischen Substrate eine Gasphase. Diese wird durch ein Rohr 40 abgeführt. Die Wärme der abgeführten Gase kann zum Vorwärmen der durch die Düsenanordnung 16 zugeführte Luft genutzt werden. Insbesondere werden die aus dem Reaktionsbehälter 12 des ersten Bioreaktors I abgeführten Gase zumindest teilweise in dem zweiten Bioreaktor II genutzt (s.u.).

Das durch die Fördereinrichtung 36 in Richtung des zweiten Bioreaktors II geförderte Substrat gelangt zusammen mit dem hinzugefügten Wasser in einen Reaktionsbehälter 42. In dem Reaktionsbehälter 42 wird das Substrat durch einen in Richtung der Pfeile 44 strömenden Substratstrom in Lösung gehalten. Hierzu wird in einem oberen Bereich des Behälters 42 Substrat entnommen und über eine Rohrleitung 46 mittels einer Pumpe 48 in einem unteren Bereich des Behälters 42 diesem wieder zugeführt. Hierbei handelt es sich um ein Kreislaufsystem. Innerhalb der Rohrleitung 46 ist ein weiterer Wärmetauscher 48 zur unmittelbaren Entnahme von Wärme aus dem Substrat vorgesehen. Der Wärmetauscher 48 kann mit dem Wärmetauscher 34 gekoppelt sein. Im Anschluss an den Wärmetauscher 48 ist in der Rohrleistung 46 eine Belüftungsund Regulierungseinrichtung 50 vorgesehen. Die Belüftungs- und Regulierungseinrichtung 50 dient zur Zufuhr von Sauerstoff zu dem Substrat sowie zur Regulierung des pH-Wertes. Über eine Zuführleitung 52, die vorzugsweise mit der Rohrleitung 40 des Bioreaktors I verbunden ist, können Abgase des Bioreaktors I unmittelbar dem in dem Bioreaktor II verarbeiteten Substrat zugeführt werden. Über eine zweite Rohrleitung 54 können die zugeführten Gase abgeleitet werden. Die durch die Rohrleitung 54 abgeleiteten Gase werden vorzugsweise einer Verbrennungseinrichtung 56 zugeführt. Das aus dem Behälter 42 im oberen Bereich entnommene Medium wird durch ein Sieb und/oder einen Filter 58 geleitet. Die Erwärmung des in dem Reaktionsbehälter 42 vorhandenen Substrats erfolgt wiederum durch mikrobielle Oxidationsvorgänge.

Der Behälter 42 ist vorzugsweise ebenfalls doppelwandig ausgebildet, so dass ein Wärmetransportmedium, wie Wasser, durch einen Hohlraum 60 transportiert und innerhalb des Behälters 42 erzeugte Wärme aufnehmen kann. Der Transport des Mediums erfolgt über Rohrleitungen 62 und eine Pumpe 64. Hierbei wird das Medium durch einen Wärmetauscher 66 zur Abfuhr von Wärme geleitet. Der Wärmetauscher 66 kann mit dem Wärmetauscher 48 und/oder dem Wärmetauscher 34 gekoppelt sein.

Vor dem Bioreaktor II wird dem aus Bioreaktor I kommenden Sediment H₂O zugesetzt. Diese "Slurry" wird in einem erneut aeroben Verfahren weiter oxidiert.

Die Temperatur im Bioreaktor soll konstant gehalten werden. Dies wird durch die Variation der Sauerstoffbeimischung im Belüftungstank (50) erreicht. Da die Wärmeentwicklung in erster Linie durch die Nutzung von O₂ als terminalem Elektronenakzeptor bedingt ist, soll auch hier die Temperatur über eine Limitierung oder Erhöhung des verfügbaren Sauerstoffs reguliert werden.

Die gewählte Betriebstemperatur in den Bioreaktoren I und II ist in beiden Fällen abhängig von der Temperaturtoleranz der eingesetzten Mikroorganismen.

Im unteren Bereich des Behälters 42 des zweiten Bioreaktors II ist wiederum eine Fördereinrichtung 68 vorgesehen, bei der es sich ebenfalls um eine Schraubenschnecke handeln kann. Durch die Fördereinrichtung 68 wird Substrat durch eine Rohrleitung 70 in Richtung des dritten Bioreaktors III gefördert. Das Substrat wird in eine Reaktionsbehälter 72 des Bioreaktors III mittels einer vorzugsweise vertikalen Düsenanordnung 74 eingebracht. Vorzugsweise enden die Düsen auf unterschiedlichen Höhen in dem Reaktionsbehälter 72. Zusätzlich erfolgt eine Durchmischung des Substrats in dem Reaktionsbehälter 72, in dem im oberen Bereich des Reaktionsbehälters 72 Substrat über eine Rohrleitung 76 entnommen, mit einer Pumpe 78 gefördert und über Düsen 80, vorzugsweise im unteren Bereich des Reaktionsbehälters 72, diesem wieder zugeführt wird.

Der Reaktionsbehälter 72 ist vorzugsweise ebenfalls doppelwandig ausgebildet, so dass in einem Hohlraum 82 Wärmetransportmedium vorgesehen ist, das über eine Pumpe 84 und Rohrleitungen 86 durch einen Wärmetauscher 88 zur Entnahme von Wärme gefördert wird. Der Wärmetauscher 88 kann wiederum mit dem Wärmetauscher 34 und/oder dem Wärmetauscher 66 und/oder dem Wärmetauscher 68 verbunden sein.

Im unteren Bereich des Reaktionsbehälters 72 ist eine weitere Fördereinrichtung 90, wie eine Schraubenschnecke, vorgesehen. Von der Fördereinrichtung 90 wird das verbleibende Restsubstrat, bei dem es sich im Wesentlichen um Humus handelt, durch eine Rohrleitung 92 abgeführt. Die bei der Fermentation in dem Bioreaktor III entstehenden Gase werden durch eine Rohrleitung 94 abgeführt und der Verbrennungseinrichtung 56 zugeführt.

Die Durchmischung des Substrats in dem Reaktionsbehälter 72 erfolgt entweder, wie vorstehend beschrieben, durch die Düsen 80 und die Pumpe 78 oder alternativ auch durch ein in dem Reaktionsbehälter 72 vorgesehenes Rührwerk.

### Erste aerobe Stufe (nachfolgend auch "ARS I"; bevorzugt für Pilze)

Der neuartige Bioreaktor zur Fermentation fester Substanzen ermöglicht die Etablierung eines Systems der kontrollierten thermischen Nutzung organischer Reststoffe. Eine gezielte Belüftung, Durchmischung und Regulation war zuvor in gerührten Bioreaktoren nicht möglich.

Die erste aerobe Stufe (ARS I) dieses Prozesses wird im kontinuierlichen Betrieb mit einem Feststoffgehalt um 70%, dass heißt ohne makroskopisch sichtbare Wasserphase betrieben.

Mit H₂O gesättigter Druckluft, die von einem Kompressor bereit gestellt wird, gelangt durch vertikale Düsen 18, die in den Bioreaktor eingefahren werden können, in den Bioreaktor und belüftet diesen gleichmäßig. Die Düsen werden durch Siebe 22 (Gitter) mit abnehmendem Ausschlussvolumen geführt. Die Gitter verhindern zum Einen eine zu feste Schüttung der organischen Reststoffe, dienen zum Anderen aber auch der homogenen Belüftung (durch verteilen und brechen der möglichen Luftblasen). Das Gittersystem kann mit Druckluftdüsen versehen sein, um ein zusätzliches gepulstes Belüften zu ermöglichen.

Anaerobe Regionen im Fermentationsgut sollen in dieser Fermentationsstufe vermieden werden, um den Methan-Gehalt in der Abluft klein zu halten. Die warme Abluft wird parallel zur Zuluftleitung abgeführt, um die Zuluft zu erwärmen (Energieerhalt). Die Abluft wird weitergeleitet durch den Belüfter in der zweiten aeroben Stufe und von da aus weiter in den Methan-Brenner nach der anaeroben Stufe III, um alle während der Fermentation gebildeten VOCs (volantile organic carbon) zu oxidieren.

Zusätzliche kurze Düsen am Deckel 10 des Bioreaktors 12 dienen der Regulation. Über die Zugabe von Wasser, möglichen Wuchsstoffen oder Puffer (bevorzugt saure Puffer, die für Pilze geeignet sind), kann auf den Fermentationsverlauf Einfluss genommen werden.

Die Temperatur im gesamten Bioreaktorsystem soll über die Druckluftversorgung und damit über die Verfügbarkeit von O₂ reguliert werden. Exotherme Reaktionen benötigen in der Regel O₂, dass heißt je weniger O₂ den aeroben Mikroorganismen in den ersten beiden Fermentationsstufen zu Verfügung steht, um so langsamer läuft ihr Stoffwechsel und um so weniger Wärmeenergie wird freigesetzt.

Der Deckel 10 mit den vertikalen Düsen kann ganz oder teilweise hochgefahren werden, um organische Reststoffe nachzufüllen und den Bioreaktor im Bedarfsfall zu reinigen. Zum Befüllen wird der Bioreaktor nur in einem kleinen Segment hochgefahren, da sonst durch die unterbrochene Belüftung anaerobe Verhältnisse entstehen können und damit die in dieser Stufe unerwünschte Methanproduktion einsetzen kann (Emissionsschutz).

Die bevorzugte Ausführung zum Nachfüllen von organischen Reststoffen ist eine Vorkammer, die durch zwei Klappen von Umgebung beziehungsweise dem Reaktor-Innerem abgeschlossen ist. Beim Öffnen der äußeren Klappe ist die innere geschlossen. Nach Einfüllen der organischen Reststoffe wird die äußere Klappe geschlossen, ein Schredder-Mechanismus in der Vorkammer setzt ein und zerkleinert und homogenisiert die Reststoffe. Ist dies geschehen, öffnet sich die innere Klappe und die organischen Reststoffe fallen in den Reaktor. Dieses Vorgehen hat den Vorteil, dass kaum Wärme und keine Gase aus dem Reaktor entweichen können.

Der gesamte Reaktor ist gut isoliert und mit einem mit Wärmemittel gefüllten Doppelmantel 26 umgeben, der an einen Wärmetauscher 34 angeschlossen ist.

Am Boden des Reaktors ist eine Schraubenschnecke 36 angebracht, die kontinuierlich den sedimentierten Bodensatz in den zweiten aeroben Bioreaktor transportiert. Optional können im Bioreaktor in verschiedenen Höhen zusätzliche Schraubenschnecken angebracht sein, die für eine zusätzliche Durchmischung sorgen.

### Zweite aerobe Stufe (nachfolgend auch "ARS II"; bevorzugt Bakterien)

Von (ARS I) wird über eine oder mehrere Schneckenschrauben Substrat herantransportiert. Vor ARS II wird der in ARS I voroxidierte und zerkleinerte organische Reststoff mit Wasser auf einen Feststoffgehalt von 40% verdünnt und in den Reaktor 12 gegeben. Bei diesem Reaktor handelt es sich um ein geschlossenes System ohne Gasphase.

Die Reststoffsuspension wird im Kreislauf gepumpt und durch den kontinuierlichen Medienstrom 44 in Schwebe gehalten. An der Oberseite des Reaktors wird die Suspension gesiebt und gefiltert, dann zur Energiegewinnung durch einen Wärmetauscher 48 geführt.

Dem Wärmetauscher nachgeschaltet ist eine Belüftungs-/ Mess- / Regeleinheit 50, in der die O₂-Konzentration gemessen und durch eine Änderung der Druckluftversorgung in ARS I reguliert wird. Bei einer Steigerung des Luftstroms in ARS I steigt der O₂-Gehalt in dessen Abluft, die im Belüfter von ARS II erneut genutzt wird. Bei einer möglichen Unterversorgung mit O₂ von ARS II, das zu einem ungewünschten Absinken der Temperatur führt, kann zusätzlich Druckluft über den ARS I vorangestellten Kompressor bereit gestellt werden.

Im Belüfter wird der pH des Mediums reguliert (bevorzugt neutral, da Bakterien). Hierzu wird die Lösung leicht alkalisiert und das während der mikrobiellen Atmung entstehende HCO₃ durch Calzium komplexiert und ausgefällt. Das ausgefällte Calzimcarbonat wird anschließend in einem Säurewäscher regeneriert, im dem das Bicarbonat als CO₂ freigesetzt wird. Die Abluft des Belüfters wird durch den Methan-Brenner des Bioreaktors III (ANRS III, s. u.) geleitet, um die bei der Fermentation entstehenden VOCs zu verbrennen.

Eine Pumpe 49 führt das mit Sauerstoff angereicherte Medium an der Unterseite wieder in den Bioreaktor ein.

Auch dieser Bioreaktor ist mit einem Doppelmantel 60, der mit einem Wärmemittel gefüllt ist, umgeben und an einen Wärmetauscher 66 gekoppelt.

Am Boden des Bioreaktors ist eine Schneckenschraube 68 angebracht, die sedimentierte Reststoffe in Bioreaktor III (ANRS III; s. u.) überführt.

### Dritte anaerobe Stufe (nachfolgend auch "ANRS III"; methanogene Bakterien)

Von ARS II wird über eine oder mehrerer Schneckenschrauben Substrat herantransportiert und durch Zumischung von Wasser auf einen Feststoffgehalt von 10% verdünnt. Die Suspension wird durch vertikal in den Reaktorraum ragende Düsen 78 eingebracht (müssen nicht, sollten aber kurz sein). Durchmischt wird der anaerobe Reaktor durch ein vertikales Düsensystem 80. An der Oberseite des Reaktors wird die Suspension abgepumpt und in Pulsen durch das Düsensystem in den Bioreaktor gedrückt.

Bei der Vergärung in diesem Reaktor wird weniger Wärme pro Volumeneinheit frei als in den aeroben ARS I und ARS II. Die Restwärme wird dennoch über einen Doppelmantel 82 mit Wärmemittel durch einen Wärmetauscher 88 genutzt. Bevorzugt soll in dieser dritten Stufe Methan produziert werden. Das Methan wird über ein Leitung 94 abgeführt, gesammelt und in einem Verbrennungsmotor 56 in dem die Abluft der beiden vorangegangenen Stufen verbrannt wird, zur Stromerzeugung genutzt.

Der hierbei erzeugte Strom soll ausreichen, um alle Energie verbrauchenden Schritte der Fermentation, wie Schredder, Pumpen, Wärmepumpen, Schraubenschnecken und den Kompressor zu energetisieren.

Am Boden des anaeroben Reaktors ist eine Schraubenschnecke 90, die den nicht zu metabolisierenden Bestandteil in Reststoffbehälter transportiert. Der Reststoff kann als Humus genutzt werden.

Das Verfahren gemäß Ausführungsform (2) der Erfindung umfasst wenigstens einen im Wesentlichen anaeroben und einen im Wesentlichen aeroben Verfahrensschritt in nacheinander geschalteten diskreten Reaktionsgefäßen. Es ist dabei bevorzugt, dass zunächst wenigstens ein anaerober Verfahrensschritt erfolgt.

In einer besonders bevorzugten Ausführungsform umfasst das erfindungsgemäße Verfahren wenigstens drei Fermentationsschritte, nämlich einen ersten aeroben Schritt (a), einen zweiten im Wesentlichen aeroben Schritt (b) und einen im Wesentlichen anaeroben Schritt (c). Aus Gründen des Geruchs ist es dabei bevorzugt, dass das Verfahren als im Wesentlichen geschlossenes System betrieben wird, wobei die Abluft des Schrittes (a) als Zuluft des Schrittes (b) dient, und wobei die Abluft des Schrittes (b) zusammen mit dem in Schritt (c) erzeugten Biogas verbrannt wird. Bei dieser Reaktionsführung ist bevorzugt, dass die ersten beiden Schritte durch die Sauerstoffzufuhr des Schrittes (a) gesteuert werden (durch die Verwendung der Abluft von Schritt (a) als Zuluft von Schritt (b) wird dadurch auch die Sauerstoffzufuhr des Schritts (b) beeinflusst).

In besonders bevorzugten Ausführungsformen werden die Reaktionsbedingungen der Schritte (a) - (c) wie folgt eingestellt:

### Schritt (a)

(i) wird mit einem Feststoffgehalt von wenigstens 50 Gew.-% betrieben; und/oder
(ii) wird bei einem pH ≤ 6, vorzugsweise einem pH von 3 bis 5, betrieben, wobei der Abbau im Wesentlichen durch Pilze (z. B. Schimmelpilze, Hefen usw.) erfolgt; und/oder
(iii) es erfolgt eine pH-Pufferzugabe, insbesondere die Zugabe eines Citratpuffers; und/oder
(iv) die Sauerstoffzufuhr ist so geregelt, dass eine Reaktortemperatur von 30 ± 15 °C herrscht.

### Schritt (b)

(i) wird mit einem Feststoffgehalt von wenigstens 25 Gew.-%, vorzugsweise von 30 bis 50 Gew.-%, betrieben; und/oder
(ii) wird bei einem pH von ≥ 7, vorzugsweise bei pH von 7 bis 9, betrieben, wobei der Abbau im Wesentlichen durch Bakterien, insbesondere Pseudomonaden, erfolgt; und/oder
(iii) es erfolgt eine pH-Pufferzugabe, insbesondere die Zugabe eines Phosphatpuffers, und/oder die Zugabe von Salzen, insbesondere CaCl₂, zur Fällung von CO₂.

### Schritt (c) ist eine obligat anaerobe Fermentationsreaktion, die

(i) mit einem Feststoffgehalt von < 20 Gew.-%, vorzugsweise von 5 bis 15 Gew.- %, betrieben wird; und/oder
(ii) bei einem neutralen pH betrieben wird, wobei der Abbau im Wesentlichen durch methanogene Bakterien erfolgt und/oder
(iii) pH-Puffer zugegeben werden.

In dem erfindungsgemäßen Verfahren ist es weiterhin bevorzugt, dass in den Schritten (a) - (c) die Reaktionswärme durch Wärmetauscher abgeführt wird und/oder dass die Abluft von Schritt (c) einem Verbrennungsmotor zugeführt wird.

Die erfinderische Vorrichtung und das erfindungsgemäße Verfahren dienen somit zur
- Nutzung der freiwerdenden Wärmeenergie biochemischer Reaktionen zum Zwecke der Energienutzung
- In Bioreaktoren
- Durch verschiedene Mikroorganismen
- In verschiedenen für bestimmte Mikroorganismen optimierte Reaktoren
- In Aeroben und anaeroben Prozessen
- In einer Verschachtelung aerober und anaerober Reaktoren
- In einem kontinuierlichen Verfahren.

Die energetische Durchführbarkeit des erfindungsgemäßen Verfahrens sowie die Dimensionierung der erfindungsgemäßen Vorrichtung wird anhand der nachfolgenden Modellrechnung näher erläutert:

### Modellrechnung

Modellrechnung für ein Einfamiliehaus mit einem Bedarf von 11000 KWh/a (Heizung/Warmwasser)
- Pro Tag sind 30 KWh erforderlich.
- Für den organischen Reststoff wird eine Zusammensetzung von Kohlenhydraten, Eiweiß und Fett im Verhältnis 2:1:1 angenommen. Der Brennwert dieser Mischung entspricht 6 KWh/kg
- Bei einem Wirkungsgrad von 100% reichen somit 5 kg Reststoff/d.
- Unter der Voraussetzung, dass die Mikroorganismen nur 60% der eingesetzten Menge Verstoffwechseln, steigt der Bedarf an Reststoff auf 8,33 kg/d.
- Unter der Voraussetzung eines 15prozentigen technischen Wirkungsgrades der Wärmeumwandlung steigt der Bedarf an Reststoffen auf 55,5 kg/d.
- Täglich fallen dabei 22,2 kg nicht umgesetzte Reststoffe an.
- ARS I, ARS II, ANRS III befinden sich bei Betrieb der Anlage in einem Fließgleichgewicht.
- Von den insgesamt 60% der verstoffwechselbaren Reststoffmenge werden 30% in ARS I, 20% in ARS II und 10% in ANRS III umgesetzt.
- Die täglichen Umsatzraten in ARS I, ARS II und ARS III entsprechen 5, 10 und 20%.

Damit ergibt sich im Fließgleichgewicht für
ARS I eine Füllmenge von ca. 1100 kg (Feststoffgehalt 70%), dies entspricht einem Reaktionsvolumen von ca. 1500 I.
ARS II eine Füllmenge von ca. 400 kg (Feststoffgehalt 40%), dies entspricht einem Reaktionsvolumen von ca. 1000 I.
ANRS III eine Füllmenge von ca. 140 kg (Feststoffgehalt 10%), dies entspricht einem Reaktionsvolumen von ca. 1400 I.

## Patentansprüche

1. Bioreaktorsystem zur Nutzung der Wärmeentwicklung biochemischer Reaktionen, insbesondere zur Warmwasser- und Stromproduktion, mit
einem Bioreaktor (I,II) zum im Wesentlichen aeroben mikrobiellen Abbau organischer Substrate,
einem Bioreaktor (III) zum im Wesentlichen anaeroben mikrobiellen Abbau organischer Substrate,
wobei die Bioreaktoren (I,II,III) nacheinander geschaltet sind, so dass den nachgeschalteten Bioreaktoren (II,III) das Abbausubstrat des vorgeschalteten Bioreaktors (I) bzw. (II) zugeführt wird, und
mindestens einem Wärmetauscher (34,48,66,88) zur Wärmeabfuhr aus den Bioreaktoren (I,II,III).

2. Bioreaktorsystem nach Anspruch 1, **dadurch gekennzeichnet, dass** zwei im Wesentlichen aerobe Bioreaktoren (I,II) vorgesehen sind, die nacheinander geschaltet sind und dass der im Wesentlichen anaerobe Bioreaktor (III) dem zweiten aeroben Bioreaktor (II) nachgeschaltet ist.

3. Bioreaktorsystem nach Anspruch 1 oder 2, **dadurch gekennzeichnet, dass** der erste Bioreaktor (I) eine, vorzugsweise vertikal angeordnete Düsenanordnung (16), insbesondere zur Sauerstoffzufuhr und/oder mehrere in Förderrichtung nacheinander geschaltete, vorzugsweise horizontal angeordnete Siebe (22), deren Maschenweite vorzugsweise in Förderrichtung abnimmt, aufweist.

4. Bioreaktor nach Anspruch 2 oder 3, **dadurch gekennzeichnet, dass** der zweite Bioreaktor (II) eine Fördereinrichtung (48) zur Erzeugung eines, vorzugsweise vertikal nach oben gerichteten Medienstroms (44) innerhalb des Bioreaktors (II) aufweist, wobei der Medienstrom durch einen Wärmetauscher (48) geführt ist.

5. Bioreaktorsystem nach Anspruch 4, **dadurch gekennzeichnet, dass** der Medienstrom durch eine Belüftungs- und Regulierungseinrichtung (50) geführt ist, in der vorzugsweise eine Sauerstoff- und pH-Wert-Regulierung erfolgt.

6. Bioreaktorsystem nach Anspruch 5, **dadurch gekennzeichnet, dass** die Belüftungs- und Regulierungseinrichtung (50) mit einem Abgasauslaß (40) des ersten Bioreaktors (I) verbunden ist.

7. Bioreaktorsystem nach einem der Ansprüche 1 - 6, **dadurch gekennzeichnet, dass** der dritte, im Wesentlichen anaerobe Bioreaktor (III) eine, vorzugsweise vertikal angeordnete Düsenanordnung (74) zum Einbringen des Abbausubstrats aus dem zweiten Bioreaktor (II) in den dritten Bioreaktor (III) aufweist und/oder eine Fördereinrichtung (78) zur Erzeugung eines Substratstroms in dem Bioreaktor (III) aufweist.

8. Bioreaktorsystem nach Anspruch 7, **dadurch gekennzeichnet, dass** die Fördereinrichtung (78) mit der Düsenanordnung (74) verbunden ist, so dass das Einbringen des Abbausubstrats aus dem zweiten Bioreaktor (II) und das aus dem dritten Bioreaktor (III) entnommene Substrat über eine gemeinsame Düsenanordnung (74) erfolgt.

9. Bioreaktorsystem nach einem der Ansprüche 2 - 8, **dadurch gekennzeichnet, dass** der dritte Bioreaktor (III) mit einer Verbrennungseinrichtung (56) zum Verbrennen von Biogas verbunden ist, wobei die Verbrennung vorzugsweise zur Energiegewinnung insbesondere zur Warmwasser- und Stromproduktion eingesetzt wird.

10. Kontinuierliches Verfahren zum mikrobiellen Abbau organischer Substrate zur Gewinnung thermischer Energie, umfassend wenigstens einen im Wesentlichen aeroben und einen im Wesentlichen anaeroben Verfahrensschritt in nacheinander geschalteten diskreten Reaktionsgefäßen.

11. Verfahren nach Anspruch 10, wobei zunächst wenigstens ein aerober Verfahrensschritt erfolgt, wobei vorzugsweise das Verfahren die folgenden Schritte umfasst:
(a) einen ersten aeroben Schritt;
(b) einen zweiten im Wesentlichen aeroben Schritt und
(c) einen im Wesentlichen anaeroben Schritt.

12. Verfahren nach Anspruch 10 oder 11, wobei das Verfahren als geschlossenes System betrieben wird und die Abluft des Schritts (a) als Zuluft des Schritts (b) dient und die Abluft des Schritts (b) mit dem in Schritt (c) erzeugten Biogas verbrannt wird und wobei vorzugsweise die Reaktion durch die Sauerstoffzufuhr des Schritts (a) gesteuert wird.

13. Verfahren nach einem oder mehreren der Ansprüche 10 bis 12, wobei
- Schritt (a)
(i) mit einem Feststoffgehalt von wenigstens 50 Gew.-% betrieben wird;
(ii) bei einem pH ≤ 6, vorzugsweise einem pH von 3 bis 5 erfolgt, wobei der Abbau im Wesentlichen durch Pilze erfolgt;
(iii) eine pH-Pufferzugabe, insbesondere die Zugabe eines Citratpuffers umfasst und/oder
(iv) die Sauerstoffzufuhr so geregelt ist, dass eine Reaktortemperatur von 30 ± 15 °C herrscht; und/oder
- Schritt (b)
(i) mit einem Feststoffgehalt von wenigstens 25 Gew.-%, vorzugsweise von 30 bis 50 Gew.-%, betrieben wird;
(ii) bei einem pH von ≥ 7, vorzugsweise bei pH von 7 bis 9, erfolgt, wobei der Abbau im Wesentlichen durch Bakterien, insbesondere Pseudomonaden, erfolgt und/oder
(iii) eine pH-Pufferzugabe, insbesondere die Zugabe eines Phosphatpuffers, und/oder die Zugabe von Salzen, insbesondere CaCl₂, zur Fällung von CO₂ umfasst; und/oder
- Schritt (c) eine obligat anaerobe Fermentationsreaktion ist, die
(i) mit einem Feststoffgehalt von < 20 Gew.-%, vorzugsweise von 5 bis 15 Gew.-%, erfolgt;
(ii) bei einem neutralen pH erfolgt, wobei der Abbau im Wesentlichen durch methanogene Bakterien erfolgt und/oder
(iii) eine pH-Pufferzugabe umfasst.
